# EUROPEAN PATENT SPECIFICATION

(11) **EP 2 122 348 B1**
(45) Date of publication and mention of the grant of the patent: **13.08.2014**
(21) Application number: 08708204.6
(22) Date of filing: 25.01.2008
(51) Int. Cl.: G01N 33/50

(54) **METHODS TO DIAGNOSE AN AUTOIMMUNE DISEASE AND TO IDENTIFY AND ISOLATE STIMULATORY MOLECULES**
VERFAHREN ZUR DIAGNOSE EINER AUTOIMMUNKRANKHEIT UND ZUR IDENTIFIZIERUNG UND ISOLIERUNG STIMULIERENDER MOLEKÜLE
PROCÉDÉS DE DIAGNOSTIC D'UNE MALADIE AUTO-IMMUNE ET D'IDENTIFICATION ET D'ISOLEMENT DE MOLÉCULES STIMULATRICES

(30) Priority: 25.01.2007 US 886651 P
(43) Date of publication of application: 25.11.2009
(73) Proprietor: Santillo, Mariarosaria, 80129 Napoli (IT); Avvedimento, Vittorio Enrico, 81100 Caserta (IT); Gabrielli, Armando, 60020 Ancona (IT); Luchetti, Michele, 60020 Ancona (IT); Paterno', Roberto, 80132 Napoli (IT); Svegliati Baroni, Silvia, 60020 Ancona (IT); Curcio, Francesco, 33010 Pagnacco (UD) (IT)
(72) Inventor: Santillo, Mariarosaria, 80129 Napoli (IT); Avvedimento, Vittorio Enrico, 81100 Caserta (IT); Gabrielli, Armando, 60020 Ancona (IT); Luchetti, Michele, 60020 Ancona (IT); Paterno', Roberto, 80132 Napoli (IT); Svegliati Baroni, Silvia, 60020 Ancona (IT); Curcio, Francesco, 33010 Pagnacco (UD) (IT)
(74) Representative: Capasso, Olga
(86) International application number: PCT/EP2008/050876
(87) International publication number: WO 2008/090213

(56) References cited:
- WO-A-2005/116657
- WO-A-2006/127695
- WO-A-2007/013124
- BARONI SILVIA SVEGLIATI ET AL: "STIMULATORY AUTOANTIBODIES TO THE PDGF RECEPTOR IN SYSTEMIC SCLEROSIS" NEW ENGLAND JOURNAL OF MEDICINE, THE, MASSACHUSETTS MEDICAL SOCIETY, WALTHAM, MA, US, vol. 354, no. 25, 22 June 2006 (2006-06-22), pages 2667-2676, XP009079568 ISSN: 0028-4793
- SVEGLIATI SILVIA ET AL: "Platelet-derived growth factor and reactive oxygen species (ROS) regulate Ras protein levels in primary human fibroblasts via ERK1/2 - Amplification of ROS and Ras in systemic sclerosis fibroblasts" JOURNAL OF BIOLOGICAL CHEMISTRY, vol. 280, no. 43, October 2005 (2005-10), pages 36474-36482, XP002476146 ISSN: 0021-9258

## Description

### TECHNICAL FIELD OF THE INVENTION

The present invention relates to a method for the diagnosis of autoimmune diseases, as for example, rheumatoid arthritis, multiple sclerosis, Graves' disease and lung fibrosis, based on the increase of more than one molecular species in a suitable cell system. Once the cell system gives a positive result, the invention refers also to the detection of stimulatory molecules, i.e. antibodies, receptor ligands, in the biological samples, or cell medium, or cell extracts using a suitable cell system.

The method of the invention was successfully used to i) detect and isolate agonistic antibodies to PDGFR in systemic sclerosis (reference 3) and ii) validate TSH agonistic antibodies in Graves' disease.

### STATE OF THE ART

The authors of the instant invention discovered a novel level of regulation of several cellular transducers in primary cells. For instance, Ras p21 and the platelet-derived growth factor (PDGF) receptor are subjected to continuous degradation *in vivo.* Reactive oxygen species (ROS) generated by cellular anabolic (reduction) or catabolic (oxidation) processes in different cellular organelles, when produced in excess via ERK1/2, inhibit proteosomal degradation and lead to the accumulation of these proteins (references 1, 2).

### DESCRIPTION OF THE INVENTION

The present data indicate that the increase of more than one of the following molecular species represents a direct measure of activation of any type of cellular receptor. When more than one of such molecular species increases in a suitable cell system, this is a signal that a receptor in that cell system is activated.

The molecular species include:
i) the small GTP-ase Ras (human Ha-Ras, SwissProt P01112 or human Ki-Ras SwissProt P01116);
ii) phosphorylated extracellular signal-regulated kinases 1-2, (SwissProt P28482, hereinafter ERK1/2) and;
iii) reactive oxygen species (hereinafter ROS)
iv) the Dual specificity NADPH oxidase, a novel discovered NADPH oxidase (DUOX human, SwissProt Q9NRD9), is expressed in thyroid, lymphocytes and neural cells;
v) alpha smooth muscle actin (α-SMA, human NM_001613.1 GI:4501882) is selectively induced by ROS 15 min to 6 h induction;
vi) c-myc (EMBL accession X66258.1) is expressed in epithelial and mesenchimal cells;
vii) PDGF receptor alpha / beta (PGFRA_HUMAN SwissProt P16234 and PGFRB_HUMAN SwissProt P09619) is expressed in mesenchimal and epithelial cells;
viii) the stress kinase jun kinase (JNK , human NM-139070, NP-62070, NP-62070, NP-00274).

The authors have tested tyrosine or G-coupled receptors and defined a precise time window of activation (5-20 min) of the species indicated above by physiological stimulators (hormones or cytokines). The time window of receptor(s) activation by pathological stimulators is almost doubled relative to physiological stimulators (15-40 min). All these markers share a common type of regulation (degradation by proteasome, rapid induction of mRNA, induction by ROS) and can be used independently depending on the cell type analyzed.

Assay of the above molecular species may be performed by means of any techniques known in the art, as i.e. by detection with specific antibodies or ligands, by chemical detection, DCF fluorescence, by detection of amplified cDNA, etc.

The authors developed and validated these assays in several cell types. The finding can be used to identify and isolate specific agonistic molecules that activate a cellular receptor. The assay can be used to detect even minimal amounts of stimulatory molecules, to then purify and characterize them.

The method may advantageously applied on modified some cell lines, to be used as suitable cell system, in function of the disease to be diagnosed. As examples: i) Human oligodendrocytes (multiple sclerosis); ii) Human neuroblastoma cells (multiple sclerosis); iii) Primary human fibroblasts (rheumatoid arthritis, lung fibrosis); iv) Human synovial cells (rheumatoid arthritis).

The method and reagents can be applied to any type of cell providing the source of the possible specific stimulator. The same assay was successfully used to detect stimulatory molecules operating on fibroblasts, thyroid cells, neurons or lymphocytes.

The cell types representing the target of the unknown stimulatory molecule are disease-specific.

The method of the invention was validated in many examples of autoimmune disease by using for each of them a suitable cell system.

It is therefore an object of the present invention a method for the diagnosis and/or prognosis of an autoimmune disease or to monitor the efficacy of a therapy for an autoimmune disease in a subject comprising:
a) incubating under appropriate conditions a suitable cell system with a suitable amount of a biological sample drawn from the subject;
b) measuring the variation of more than one molecular species in said suitable cell system with respect to a proper control, said molecular species belonging to the following group: ROS, Ha-Ras, Ki-Ras, ERK1/2, JNK, DUOX and α-SMA,
wherein the autoimmune disease is selected from the group of Graves' disease or rheumatoid arthritis and
- when the autoimmune disease is Graves' disease, the biological sample is blood serum and the suitable cell system is a thyroid derived cell and
- when the autoimmune disease is rheumatoid arthritis, the biological sample is blood serum and the suitable cell system is a fibroblast cell system.

In the case of the diagnosis and/or prognosis of an autoimmune disease, the measured variation of more than one molecular species in said suitable cell system with respect to a proper control is an increase. In the case of the monitoring of the efficacy of a therapy for an autoimmune disease, the measured variation of more than one molecular species in said suitable cell system with respect to a proper control is a decrease.

It is understood that a positive result is obtained when an increase in more than one molecular species belonging to the following group: ROS, Ha-Ras, Ki-Ras, ERKI/2, JNK, DUOX and α-SMA with respect to a proper control is measured in suitable cell system tested with the biological samples.

The expert in the field shall select the proper biological sample and cell system in function of the autoimmune disease.

The skilled person in the art will also use a suitable cell system that is genetically modified to express a detectable marker under the control of a promoter, said promoter being inducible by one of the molecular species as belonging to the following group: ROS, Ha-Ras, Ki-Ras, ERK1/2, JNK, DUOX and α-SMA. Preferably, the detectable marker is a luciferase. Still preferably the promoter is the collagen alpha 2 promoter. Such genetically modified cell systems represent a cell system for easy and fast detection of ROS since ROS and ERK induction are indirectly measured by luminometry.

The expert in the field shall also select the proper control that may be a biological sample from a normal healthy subject, a biological sample from an affected subject before starting a therapy or after suitable time points after the start of the therapy, a biological sample from an affected subject treated at various doses of the therapeutic agent.

It is a further object of the invention a method of screening for autoimmune disease therapeutic agents comprising:
a) incubating under appropriate conditions a suitable cell system with a suitable amount of a biological sample drawn from an affected subject in the presence of a suitable amount of the putative autoimmune disease therapeutic agent;
b) measuring the decrease of more than one molecular species in said suitable cell system with respect to a proper control, said molecular species belonging to the following group: ROS, Ha-Ras, Ki-Ras, ERK1/2, JNK, DUOX and α-SMA,
wherein the autoimmune disease is selected from the group of Graves' disease, systemic sclerosis or rheumatoid arthritis and
- when the autoimmune disease is Graves' disease, the biological sample is blood serum and the suitable cell system is a thyroid derived cell;
- when the autoimmune disease is systemic sclerosis, the biological sample is blood serum and the suitable cell system is a fibroblast cell system or a Fα fibroblast;
- when the autoimmune disease is rheumatoid arthritis, the biological sample is blood serum and the suitable cell system is a fibroblast cell system.

The skilled person in the art will also use a suitable cell system that is genetically modified to express a detectable marker under the control of a promoter, said promoter being inducible by one of the molecular species as belonging to the following group: ROS, Ha-Ras, Ki-Ras, ERK1/2, JNK, DUOX and α-SMA. Preferably, the detectable marker is a luciferase. Still preferably the promoter is the collagen alpha 2 promoter. Such genetically modified cell systems represent a cell system for easy and fast detection of ROS since ROS and ERK induction are indirectly measured by luminometry.

The expert in the field shall also select the proper control that may be a biological sample from a normal healthy subject, a biological sample from an affected subject incubated in the presence of a suitable amount of an inert vehicle or excipient, a biological sample from an affected subject incubated in the presence of a suitable amount of a reference active compound or a biological sample from an affected subject incubated in the presence of a various amount of the putative autoimmune disease therapeutic agent.

The invention will be now described by way of non limiting examples referring to the following figures:

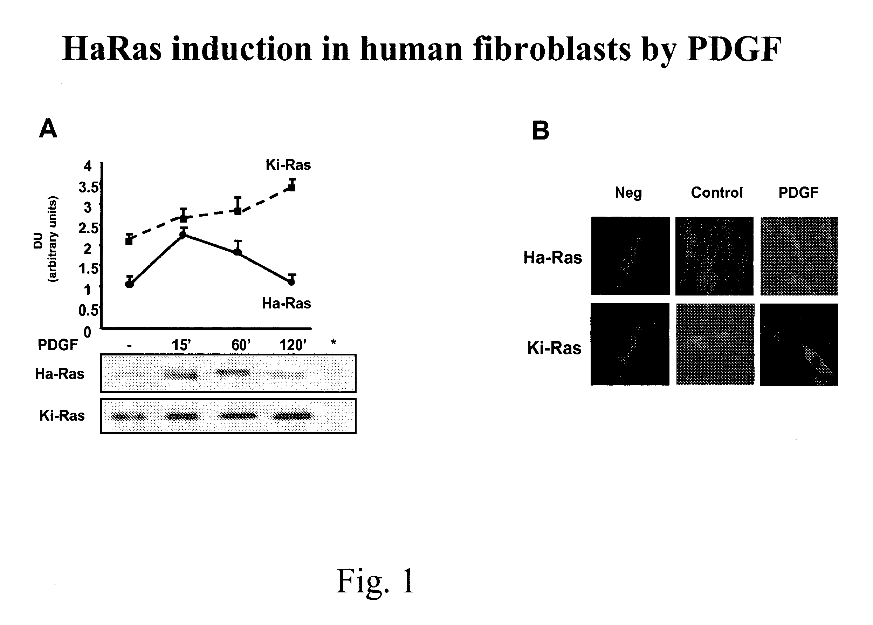

Figure 1: Induction of Ha-Ras protein by PDGF in human primary skin fibroblasts. Primary fibroblasts were incubated in 0.2% FBS for 48h and then stimulated with PDGF 15ng/ml for the indicated time. A. Total protein were extracted, immunoprecipitated with pan-Ras antibodies and immunoblotted with specific antibodies against Ha-Ras and Ki-Ras. The immunoblot shown is representative of three experiments performed in duplicate. *DU* on the ordinates indicates an arbitrary densitometric unit, normalized to the band of Ha-Ras without treatment with PDGF set to 1. **B.** fluorescence microscopy of fibroblasts preincubated in 0.2% FBS for 48 h and then stimulated with PDGF 15ng/ml for 15min. Representative results from 1 of 5 experiments are shown. *Neg* indicates immunofluorescence with non-immune sera. x40 magnification.

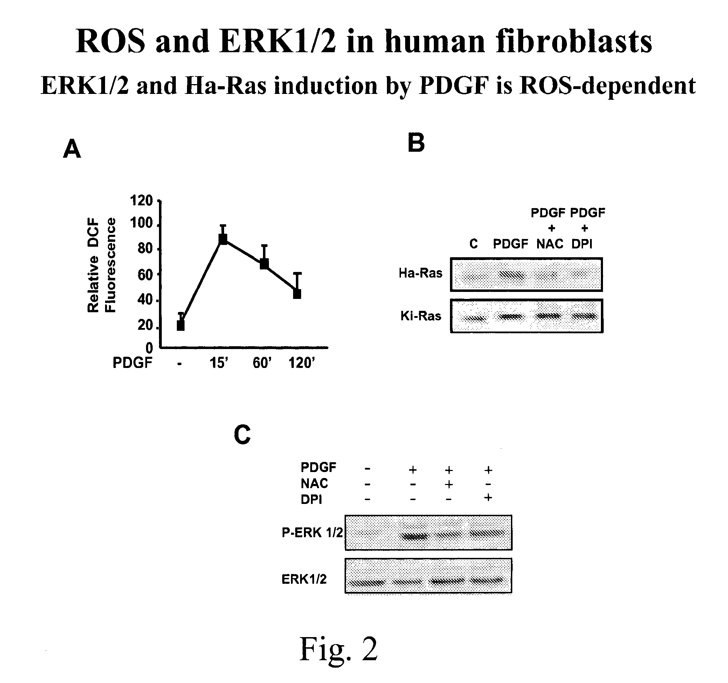

**Figure 2****:** Link between ROS-RAS and ERK1/2 and PDGFR activation and ROS inhibition leads to suppression of PDGFR, RAS and ERK1/2 activation. **A.** *Time course of ROS induction by PDGF.* Primary skin fibroblasts were incubated in 0.2% of FCS for 48 h and then stimulated with PDGF for the indicated time. ROS were determined by DCF fluorescence in normal fibroblasts in the presence or absence of PDGF (15 ng/ml). Values represent mean ± 1SD. **B**. *ROS inhibition abolishes induction of Ha-Ras by PDGF.* Primary fibroblasts were treated with NAC (20 mM 5 h) and DPI (20 uM for 1 h), treated with PDGF (15 ng/ml for 15 min) and analyzed for Ha and Ki-Ras levels as indicated in Fig. 2A. **C.** *ROS mediate PDGF induction of ERK1*/*2.* Fibroblasts incubated in 0.2% FCS for 48h, pretreated with NAC (20mM for 5h), and DPI (20uM for 1h) were treated with PDGF (15ng/ml for 30min) and analyzed for P-ERK1/2 levels. A representative of three experiment is shown.

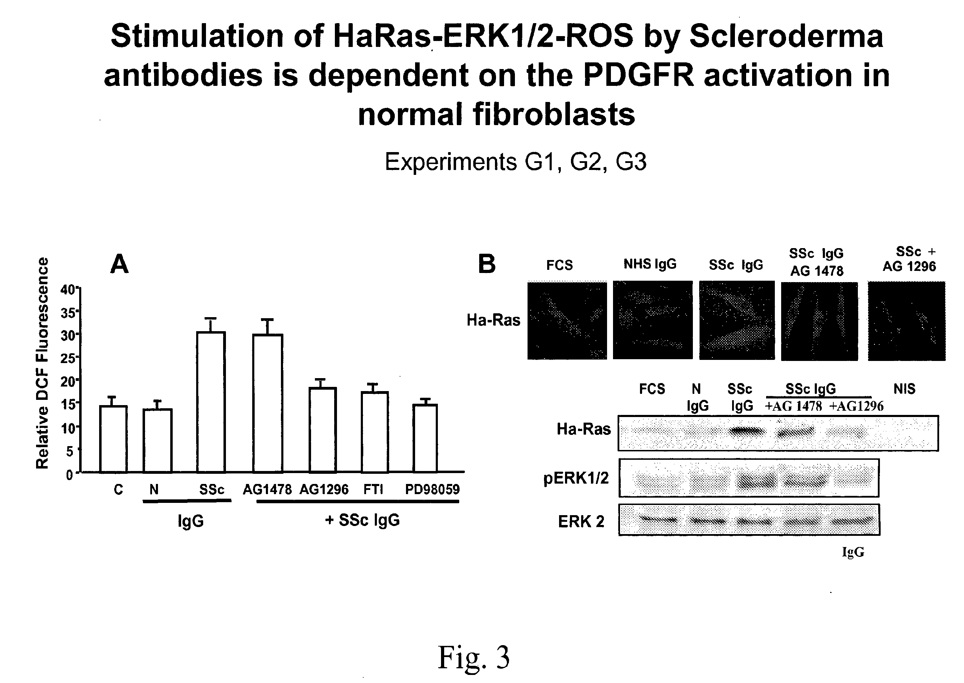

**Figure 3****:** Stimulation of Ha-Ras-ERK1/2-ROS signalling by Scleroderma antibodies is dependent on the PDGFR activation in normal fibroblasts. **A.** Levels of ROS, evaluated as the mean (+/-SE) DCF fluorescence intensity, in normal human fibroblasts incubated with IgG from three normal control (N) and IgG from three patients with systemic sclerosis (SSc) (at 200ug per ml for 15min) and preincubated with selective inhibitors of epidermal growth factor receptor (EGFR) and PDGFR (AG1478 and AG1296, re3spectively; 2uM for 2 h) and with FTI-277 (20uM for 2h) and PD98059 (40uM for 2h). **B.** fluorescence microscopical images and immunoprecipitation immunoblots for Ha-Ras protein in normal human fibroblasts incubated with normal (N) and sclerodermal (SSc) IgG (200ug/ml for 15min) in the presence and absence of selective inhibitors as in A. Representative results from 1 of 5 experiments are shown. NIS denotes non-immune serum.

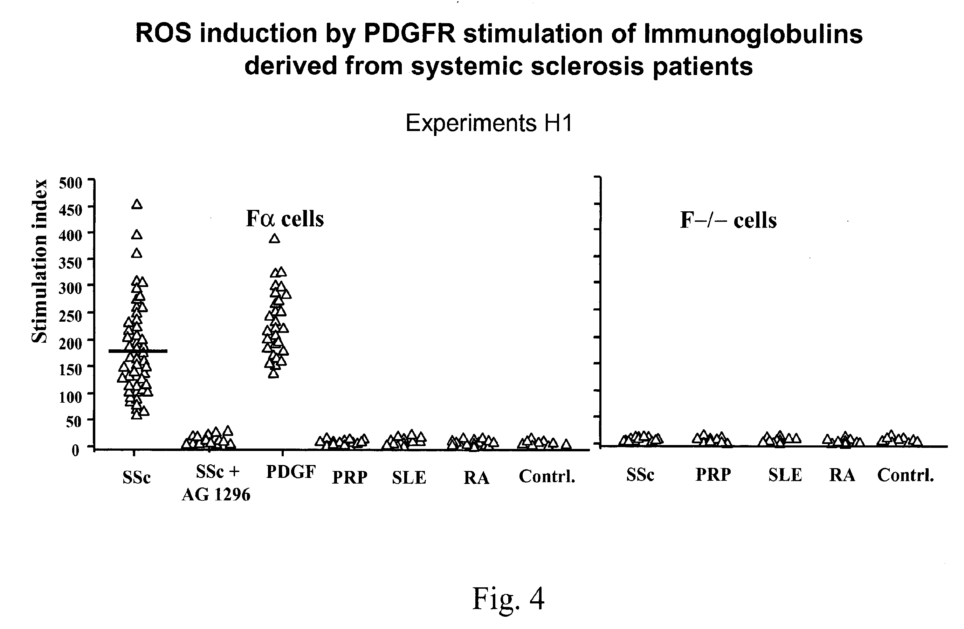

**Figure 4****:** ROS induction by immunoglobulins derived from systemic sclerosis patients is dependent on PDGFR. Levels of ROS (indicated as stimulation index) in Fα and F-/- cells incubated with normal IgG (N) and IgG from 46 SSc patients, 15 with primary Raynaud's phenomenon (PRP), 14 with systemic lupus erythematosus (SLE), 15 with rheumatoid arthritis (RA), and 10 with interstitial lung diseases (ILD) (200ug/ml for 15 min). Fα cells were also pre-incubated with a selective inhibitor of PDGF tyrosine kinase (AG1296; 2uM for 2h).

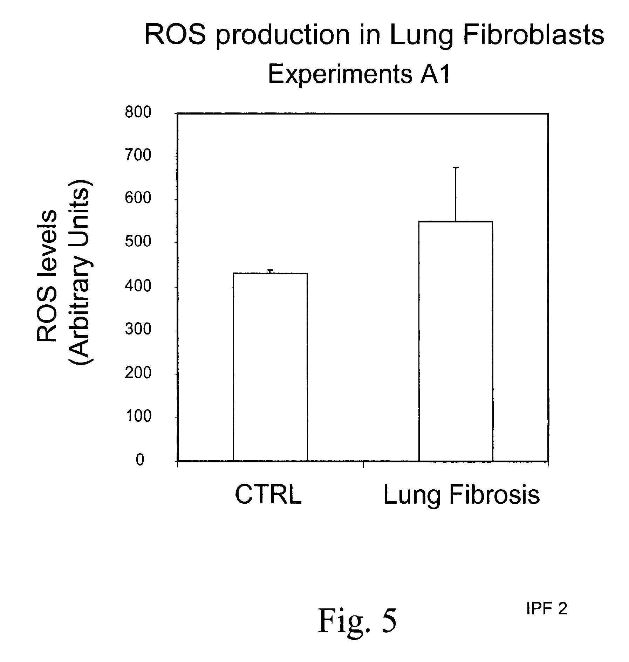

**Figure 5****:** Lung fibroblasts from patients (n=7) with idiopathic pulmonary fibrosis (LF or ILF) contain significantly higher ROS levels than normal fibroblasts. Fluorimetric measure of ROS was carried out in fibroblasts from Controls or Lung fibrosis fibroblasts at 6° passage, after loading the cells with the fluorescent probe DCHF-DA. Each sample was tested in triplicate.

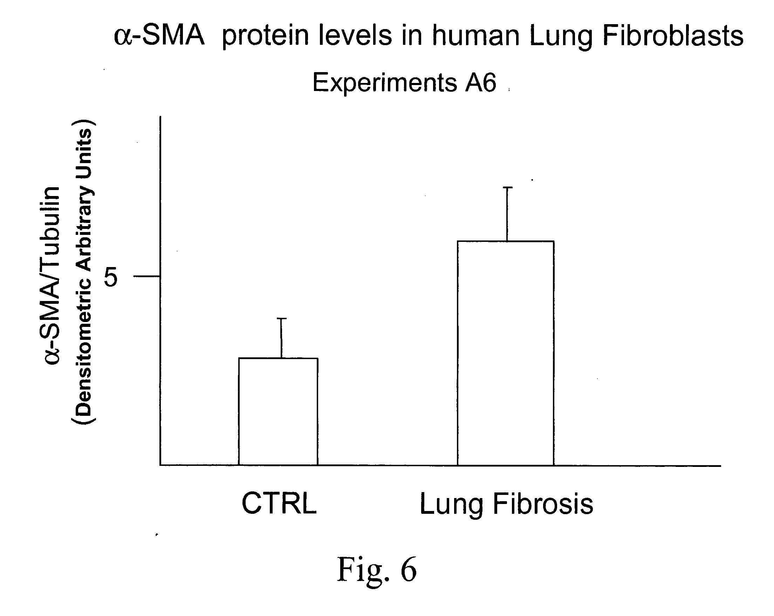

**Figure 6****:** Lung fibroblasts from patients (n=7) with idiopathic pulmonary fibrosis (LF or ILF) contain significantly higher α-SMA (accession number: NM_001613.1, GI:4501882) than normal fibroblasts. α-SMA protein levels were measured by western blot in fibroblasts from control (CTRL) or lung fibrosis fibroblasts at 6° passage.

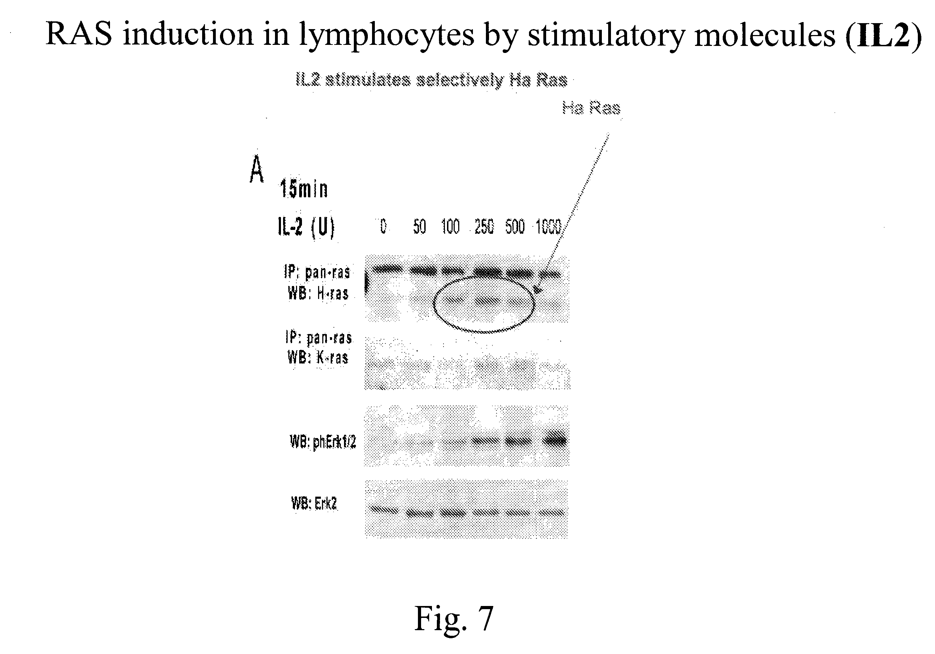

**Figure 7****:** Induction by Interleukin 2, IL-2, of Ha-Ras and ROS. The mechanism is the same as described in Fig.1 and 2. IL-2 selectively stimulates Ha-Ras and ERK1/2 in human lymphocytes. Peripheral human lymphocytes (1 x 10⁶) were stimulated with Il2 (1 U /ml) for 15 min and Ha-Ras was measured as indicated in Fig.1

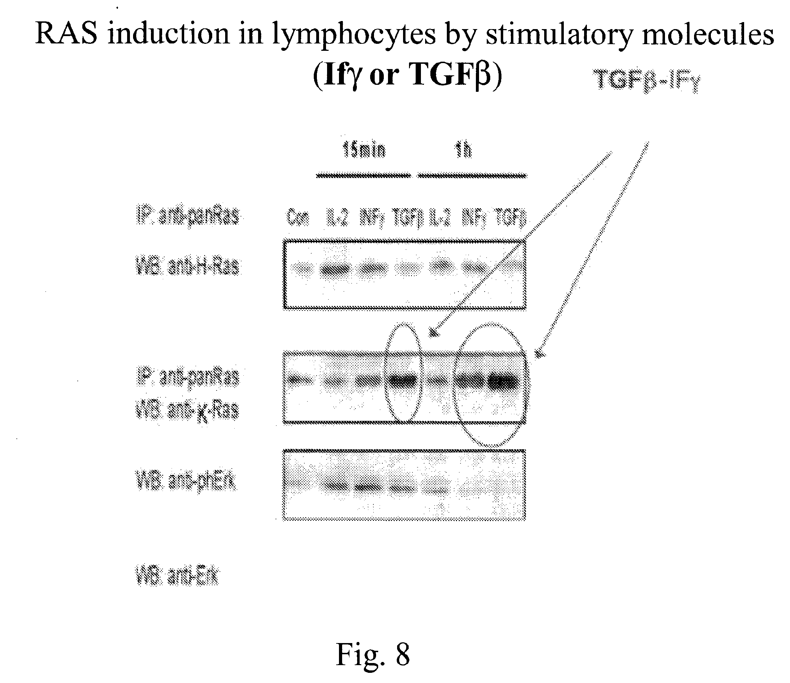

**Figure 8****:** TGF-βinduces another Ras isoform (Ki-Ras). The Ras isoform activated depends on the cell type and on the type of stimulus (see Fig. 5). Peripheral human lymphocytes (1 x 10⁶) were stimulated with Tgfb (1 ug /ml) for 15 min and Ha-Ras was measured as indicated in Fig.1.

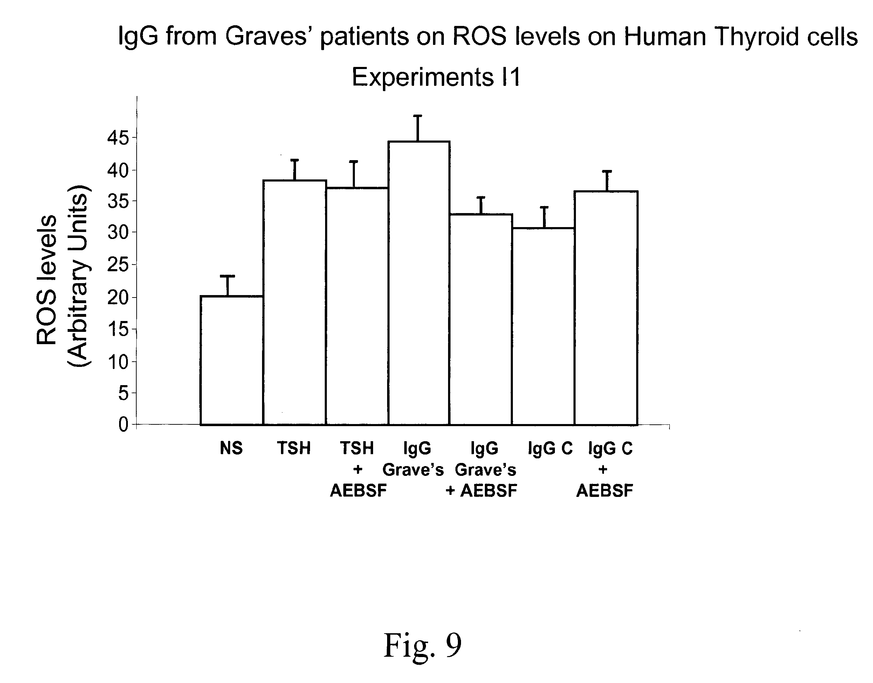

**Figure 9****:** IgG from Graves' patients (n=6) on ROS levels on Human Thyroid cells. Cells were stimulated for 15 min at 37°C with 200ug/ml of Controls (C) and Grave's patients serum IgG or TSH in the presence and absence of the NADPH oxidase inhibitor AEBSF. Fluorimetric measure of ROS was carried out after loading the cells with the fluorescent probe DCHF-DA. Each sample was tested in triplicate.

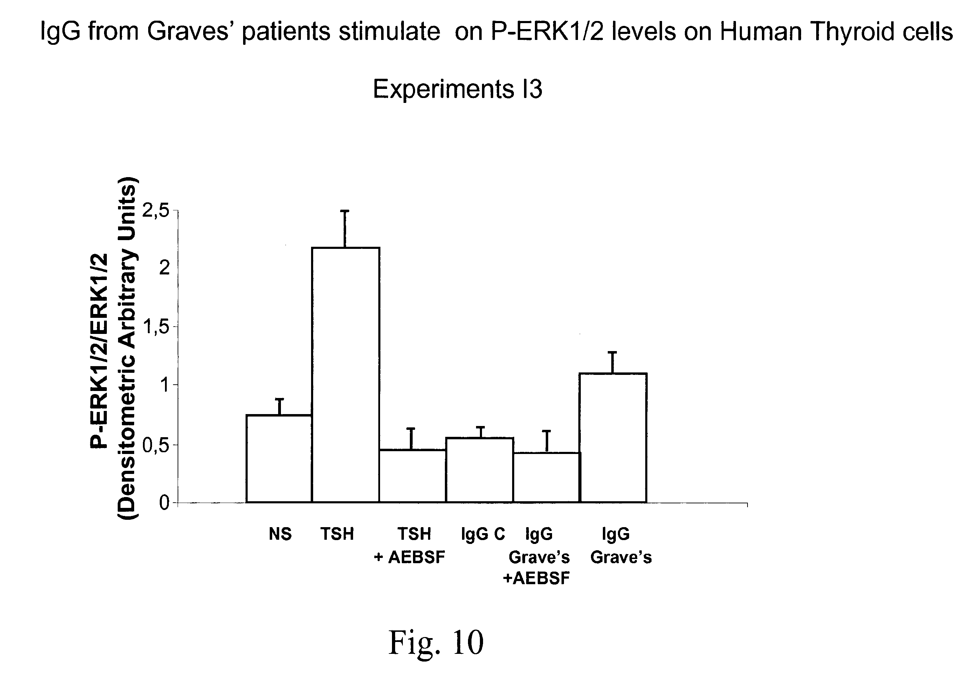

**Figure 10****:** IgG from Graves' patients (n=6) stimulate on P-ERK1/2 levels on Human Thyroid cells. Cells were stimulated for 15 min at 37°C with 200ug/ml of Controls (C) and Grave's patients serum IgG or TSH in the presence and absence of the NADPH oxidase inhibitor AEBSF. Then, cells were analyzed for P-ERK1/2 levels by Western blotting and normalized for protein loading with an anti total ERK1/2 antibody. The histogram shows the mean +/- SD values obtained by densitometric analysis of protein bands from 3 independent experiments.

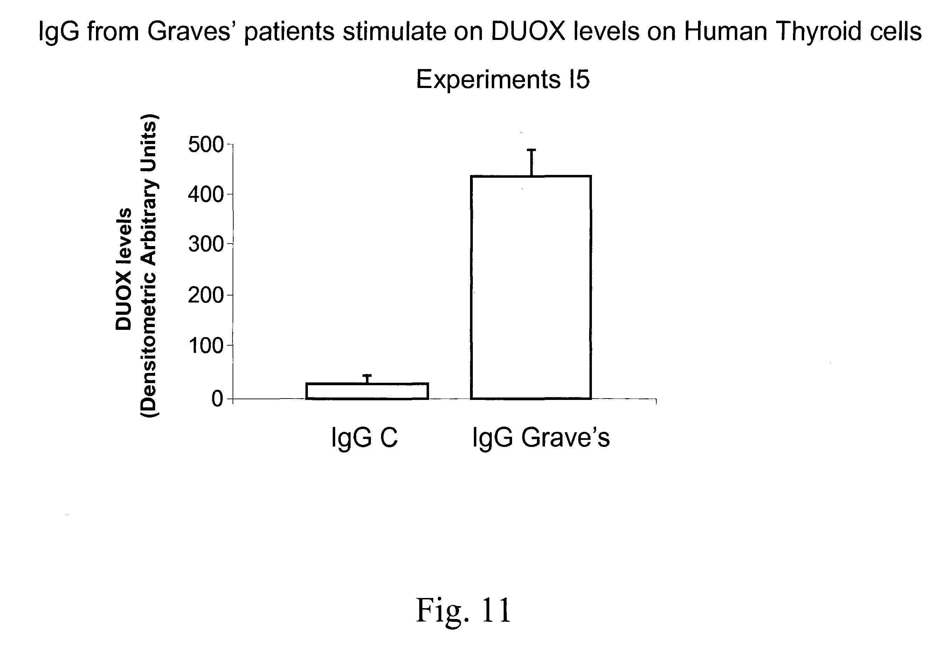

**Figure 11****:** IgG from Graves' patients (n=6) stimulate on DUOX levels on Human Thyroid cells. Cells were stimulated for 15 min at 37°C with 200ug/ml of Controls (C) and Grave's patients serum IgG Then, cells were analyzed for DUOX levels by Western blotting. The histogram shows the mean +/- SD values obtained by densitometric analysis of protein bands from 3 independent experiments.

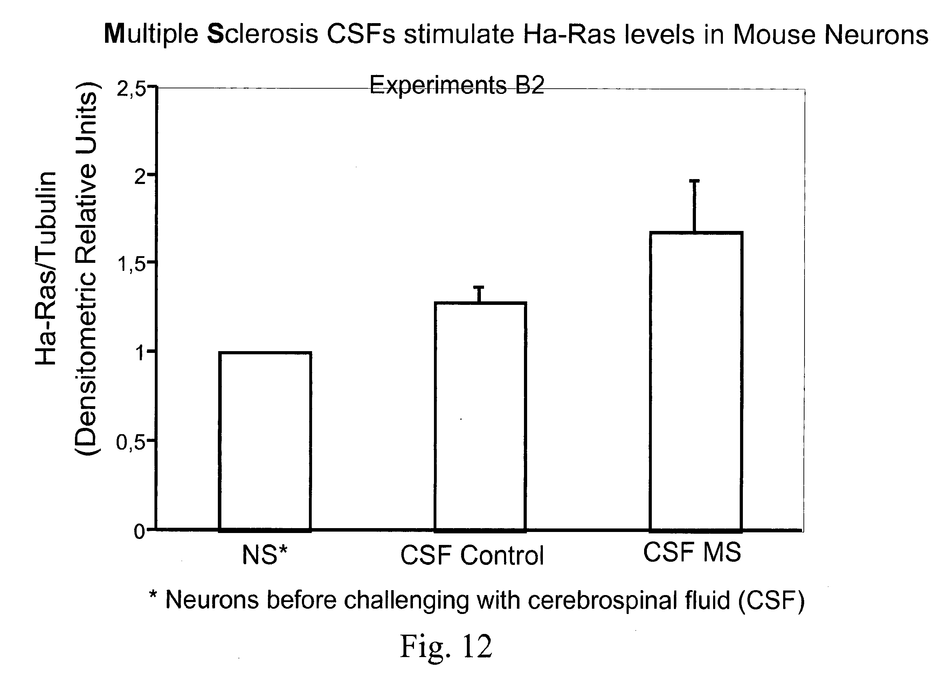

**Figure 12****:** Multiple Sclerosis CSFs (n=6) stimulate Ha-Ras levels in Mouse Neurons. Cells were stimulated for 30 min at 37°C with 30% Liquor from Control and Multiple Sclerosis (MS) patients in medium without serum. Then, cells were analyzed for Ha-Ras levels by Western blotting and normalized for protein loading with an anti α-tubulin antibody. The histograms show the mean +/- SD values obtained by densitometric analysis of protein bands from 3 independent experiments.

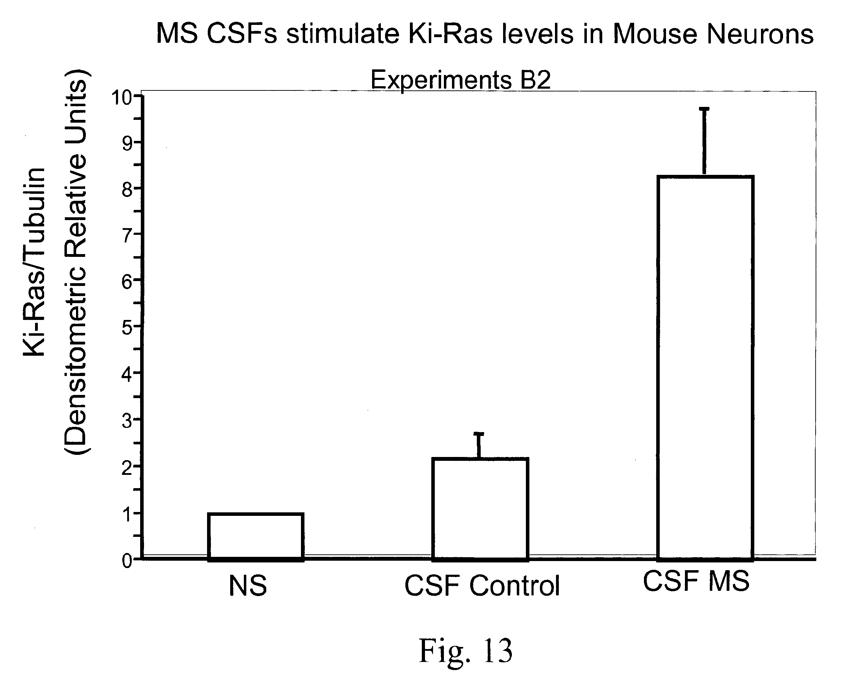

**Figure 13****:** Multiple Sclerosis CSFs (n=6) stimulate Ki-Ras levels in Mouse Neurons. Cells were stimulated for 30 min at 37°C with 30% Liquor from Control and Multiple Sclerosis (MS) patients in medium without serum. Then, cells were analyzed for Ki-Ras levels by Western blotting and normalized for protein loading with an anti α-tubulin antibody. The histograms show the mean +/- SD values obtained by densitometric analysis of protein bands from 3 independent experiments.

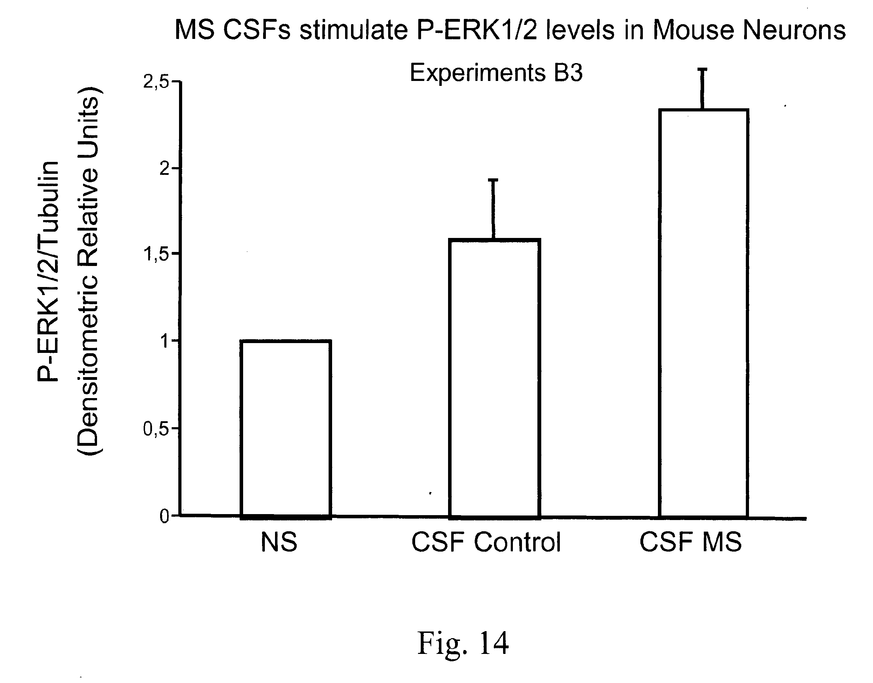

**Figure 14****:** Multiple Sclerosis CSFs (n=6) stimulate P-ERKI/2 levels in Mouse Neurons. Cells were stimulated for 30 min at 37°C with 30% Liquor from Control and Multiple Sclerosis (MS) patients in medium without serum. Then, cells were analyzed for P-ERKI/2 levels by Western blotting and normalized for protein loading with an anti α-tubulin antibody. The histograms show the mean +/- SD values obtained by densitometric analysis of protein bands from 3 independent experiments.

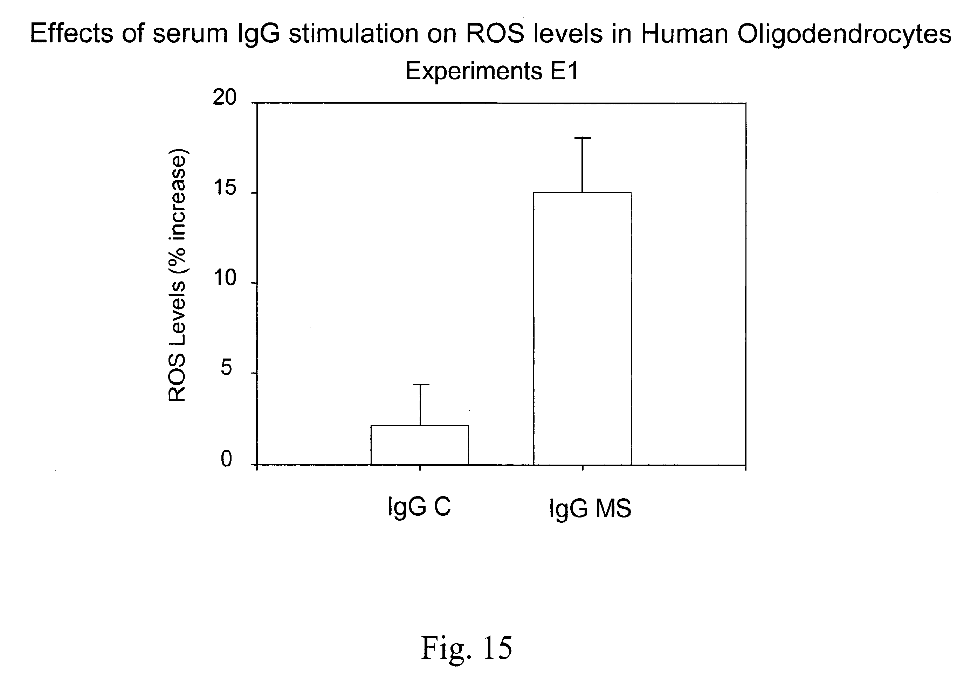

**Figure 15****:** Multiple Sclerosis serum IgGs stimulate ROS levels in Human Oligodendrocytes. Cells were differentiated for 4 days in medium without serum in presence of 100nM PMA. Then, they were stimulated for 15 min with 200ug/ml of human IgG from controls and MS patients before loading DCF for ROS measurement. Data are expressed as percent of induction over not stimulated sample.

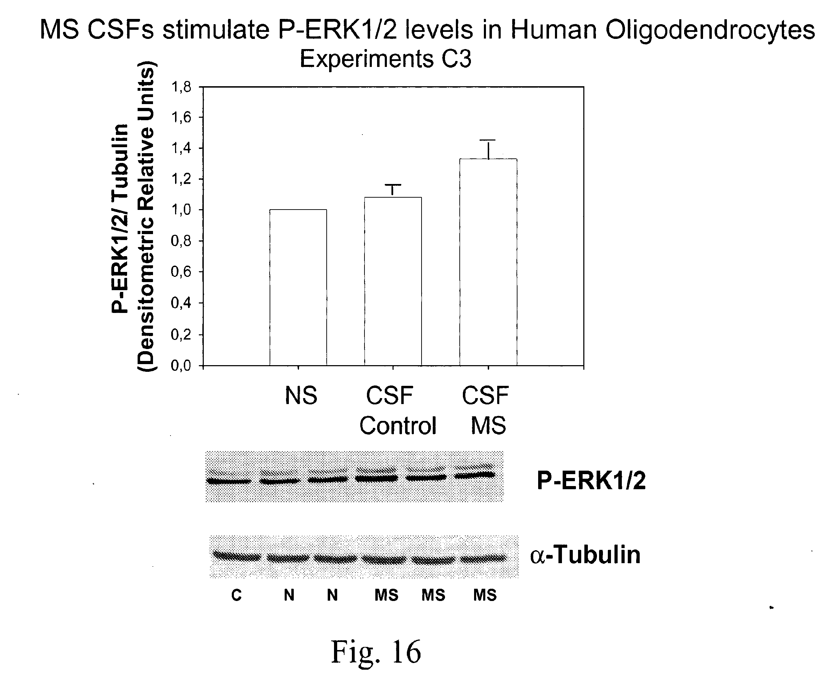

**Figure 16****:** Multiple Sclerosis CSFs (n=6) stimulate P-ERK1/2 levels in Human Oligodendrocytes. Cells were stimulated for 30 min at 37°C with 30% Liquor from Control and Multiple Sclerosis (MS) patients in medium without serum. Then, cells were analyzed for P-ERK1/2 levels by Western blotting and normalized for protein loading with an anti a-tubulin antibody. The histograms show the mean +/- SD values obtained by densitometric analysis of protein bands from 3 independent experiments. Data are expressed as relative units with not stimulated sample (C) set to 1.

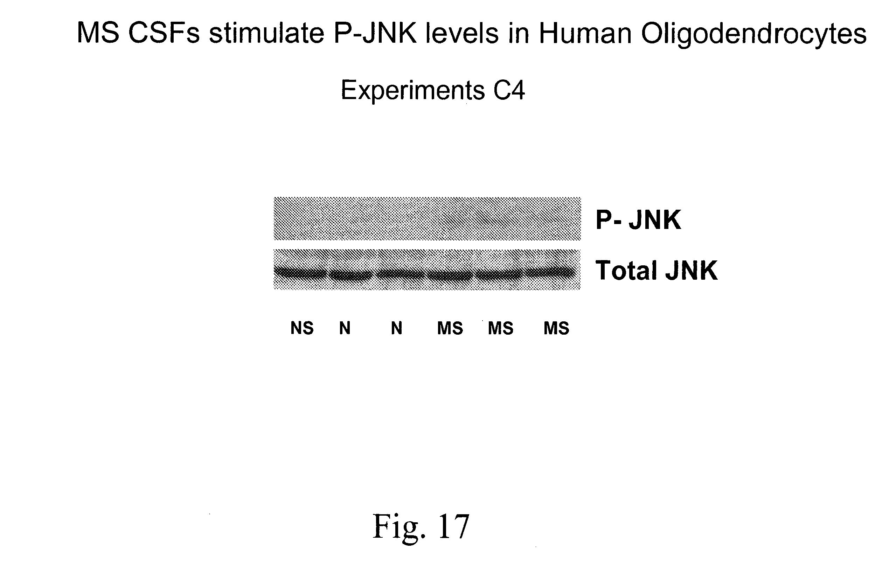

**Figure 17****:** Multiple Sclerosis CSFs (n=6) stimulate P-JNK levels in Human Oligodendrocytes. Cells were stimulated for 30 min at 37°C with 30% Liquor from Normal (C) and Multiple Sclerosis (MS) patients in medium without serum. Then, cells were analyzed for P-JNK levels by Western blotting; the same membrane was stripped and reprobed with a total JNK antibody. NS, not stimulated sample. The figure shows a representative experiment of 3.

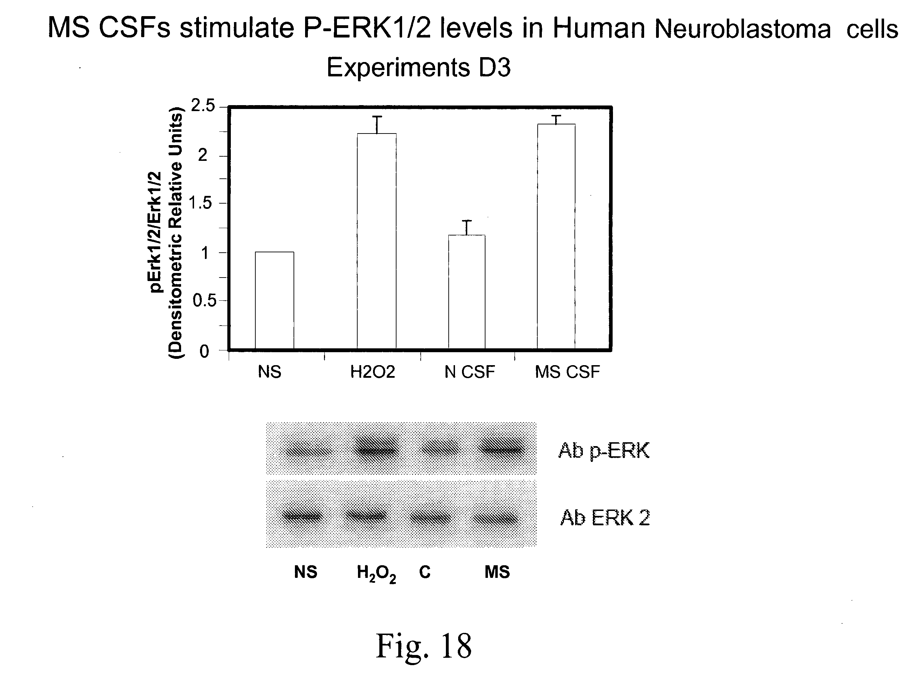

**Figure 18****:** Multiple Sclerosis CSFs (n=6) stimulate P-ERK1/2 levels in Human Neuroblastoma cells. Cells were stimulated for 30 min at 37°C with 30% Liquor from Control and Multiple Sclerosis (MS) patients or H2O2 in medium without serum. Then, cells were analyzed for P-ERK1/2 levels by Western blotting and normalized for protein loading with an anti total ERK1/2 antibody. The histogram shows the mean +/- SD values obtained by densitometric analysis of protein bands from 3 independent experiments. Data are expressed as relative units with not stimulated sample (NS) set to 1.

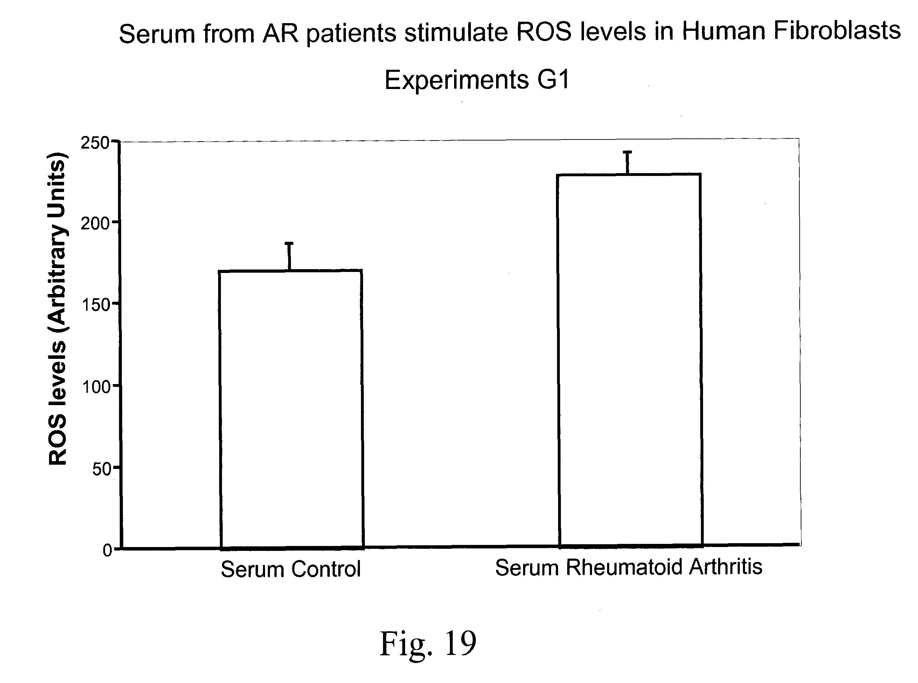

**Figure 19****:** Serum from AR patients (n=11) stimulate ROS levels in Human skin Fibroblasts. Cells were incubated in medium containing 0.2% FBS for 16 hours and then stimulated for 15min at 37°C with 20% Serum of Controls and Rheumatoid Arthritis patients. Fluorimetric measure of ROS was carried out after loading the cells with the fluorescent probe DCHF-DA. Each sample was tested in triplicate.

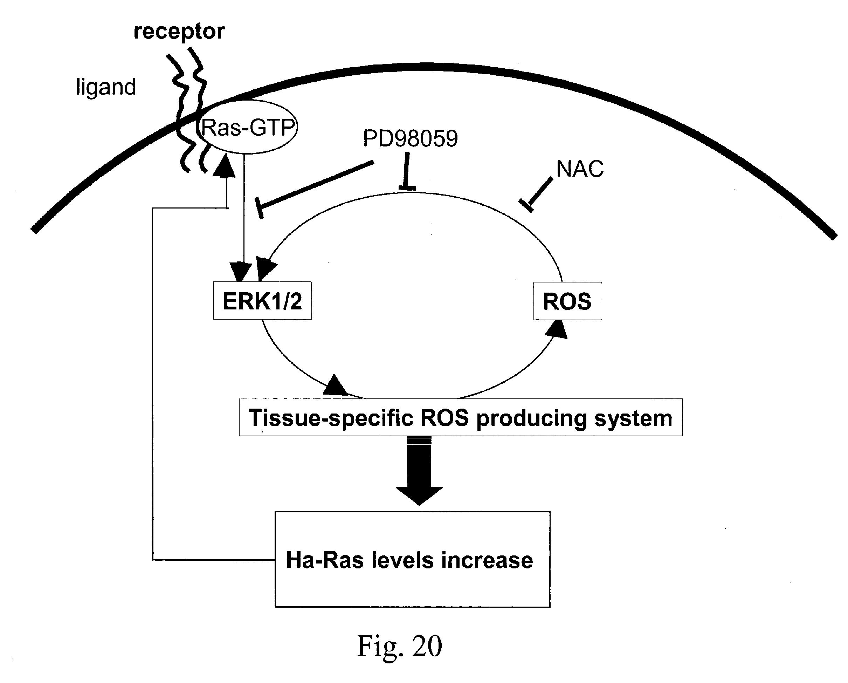

**Figure 20****:** General scheme that explains the biological basis of the methodology. The schematic diagram illustrates the circuitry initiated by PDGF and triggering ROS production by ROS producing systems. ROS activate ERK1/2, which induce Ha-Ras. Once triggered, the circuitry becomes independent from receptor signalling. The cells and the specific disease associated are indicated in each example.

### MATERIAL AND METHODS

### Cells

### Mouse cortical neurons

Cortical neuronal cultures were prepared from fetal mice (14-16 days gestation). Embryos were decapitated, and the neocortices were dissected, dissociated, and plated in Eagle's minimal essential medium (GIBCO, Auckland, New Zeland) supplemented with 20 mmol glucose (Sigma Chemical Co, St. Louis, MO, USA), 2 mmol glutamine (GIBCO, Auckland, New Zeland), 5% fetal bovine serum (GIBCO, Auckland, New Zeland) and 5% horse serum (GIBCO, Auckland, New Zeland) on culture plates which were prepared by incubation overnight with 40 ug/ml poly-D-lysine (Sigma-Aldrich, St. Louis, MO, USA) and subsequent rinsing with water. Glial cell replication was halted at days *in vitro* (DIV) 5, by 2 days exposure to 10 µmol cytosine arabinoside. Cultures were kept in a 5% coy and 95% air atmosphere at 37° C; at DIV 7 they were shifted into a growth medium identical to the plating medium, but lacking foetal serum. Cultures were used for experiments at DIV 13-14.

### Human oligodendrocyte

MO3.13 ( CELLution Biosystem Inc. , Toronto, Canada), an immortal human-human hybrid cell line that express phenotypic characteristics of primary oligodendrocytes, was created by fusing a 6-thioguanine-resistant mutant of the human rhabdomyosarcoma RD with adult human olygodendrocytes. M03-13 cells expressed surface immunoreactivity for galactosyl cerebroside (GS) and intracellular immunoreactivity for myelin basic protein (MBP), proteolipid protein (PLP), and 2', 3'-cyclic nucleotide 3'-phosphodiesterase (CNPase).

Cells were grown in Dulbecco's Modified Eagles Medium (DMEM), containing 4.5g/L glucose, supplemented with 10% Foetal Calf Serum (FCS; Sigma), 100 U/ml penicillin and 100 µg/ml streptomycin in a 5% (v/v) CO2 incubator at 37°C. Differentiated cells were prepared by first washing semiconfluent cells in DMEM-lacking serum, then culturing in DMEM supplemented with 100 U/ml penicillin, 100 µg/ml streptomycin, and 100 nM 4-β-phorbol 12-myristate 13-acetate (PMA) for 3 days prior to the experiments.

### Neuroblastoma cells

Human neuroblastoma SK-N-BE cells [American Type Culture Collection (ATCC), LGC-Promochem Teddington, Middlesex, UK] were grown in monolayer in RPMI 1640 medium (Sigma Chemical Co, St. Louis, MO, USA) supplemented with 10% foetal bovine serum (FBS; Sigma Chemical Co, St. Louis, MO, USA), 2 mM L-glutamine, 50µg/ml streptomycin and 50 IU/mL penicillin; the cells were kept in a 5% CO₂/95 air atmosphere at 37°C.

### Human skin fibroblasts

Human skin fibroblasts were obtained from punch biopsies taken from the forearms of normal volunteers were grown in Dulbecco's Modified Eagles Medium (DMEM), containing 1 g/L glucose, supplemented with 10% Foetal Calf Serum (FCS; Sigma), 100 U/ml penicillin and 100 µg/ml streptomycin in a 5% (v/v) CO2 incubator at 37°C. Fibroblasts between the fourth and the sixth subpassage were used for all experiments.

### Mouse embryo fibroblasts PDGFR -/-

Mouse embryo fibroblasts derived from PDGF receptor knock-out embryos that do not express α and β chains PDGFR subunits (F-/- cells) and F-/- cells infected with PDGFR α (Fα) have been described elsewhere (reference 4).

### Lung fibroblast, thyroid cells and lymphocytes

Lung fibroblasts were cultured according to the method indicated in reference 3, thyroid cells were cultured according to the method of reference 5. Lymphocytes were buffy coat preparations.

### Engineered cell lines

Clones of human oligodendrocytes, neuroblastoma cells and mouse MEF expressing the col2 promoter (sequence indicated in reference 6) driving the expression of firefly luciferase gene (sequence indicated in reference 7), have been generated and characterized for ROS and ERK1/2 induction. Such cells represent a cell system for easy and fast detection of ROS. As a matter of fact, ROS and ERK induction are indirectly measured by luminometry.

### Patients

### Systemic sclerosis patients

The recruitment of these patients has been described in reference 3.

### Grave's patients

The recruitment of these patients was performed following the criteria described in reference 8.

### Multiple sclerosis patients

Men and women 18 to 50 years of age with a diagnosis of relapsing MS, an EDSS store between 0 and 5.0, an MRI scan demonstrating lesions consistent with MS, and at least one medically documented relapse swithin the 12 months before randomization were included in the study. Healthy controls used were sex and age matched with MS patients.

### Rheumatoid arthritis patients

Men and women 40 to 70 years of age with a diagnosis of Rheumatoid Arthritis were included in the study. Healthy controls used were sex and age matched with AR patients. All human studies were approved by the institutional ethics committee.

### Reactive oxygen species measurement

Measurement of ROS generated by cells was carried out after loading the cells with 10µM 2',7'-dichlorofluorescein diacetate (DCFH-DA, Molecular Probes, PoortGebouw, The Nedherlands) for 10min at 37°C in medium without serum followed by two washes in PBS using the plate fluorimeter, Fluoroskan Ascent FL (Thermo Spectronics). Each sample was tested in triplicate. The intra-plate variation was less than 3 percent.

### Western Blotting

Cells were washed twice with PBS and harvested in cold RIPA buffer containing 2.5mM Na-pyrophosphate, 1mM β-glycerophosphate, 1mM NaVO₄, 1mM NaF, 0.5mM PMSF, and a cocktail of protease inhibitors.

Protein content of total lysates was determined according to method of Lowry.

Fifty micrograms of proteins from total lysates were electrophoresed on SDS-PAGE, transferred to nitrocellulose membrane and blocked in 5 % TBS-milk and 0.1 % Tween 20 for 1 h at room temperature. The filters were then probed with primary antibodies following manifacturer's instructions. The primary antibodies Ha-Ras, Ki-Ras, p-ERK1/2, Total ERK1/2, P-JNK, total JNK and were from Santa Cruz Biotechnology, anti b-Tubulin were from Sigma, anti α- SMA was from S. Cruz and anti DUOX antibody was obtained by immunization of rabbit with a peptide corresponding to amino acid x-x (not shown). Protein bands were revealed by ECL and quantified by densitometry using Scion Image software.

### Serum IgG purification

IgG fractions were purified by affinity chromatography on protein A/G sepharose column (Pierce, Rockford, IL) from serum of normal subjects and Multiple Sclerosis or Grave's disease patients. Protein concentrations were estimated by spectrophotometry.

### Drug treatment

N-acetyl-L-cysteine (NAC, Sigma, St Louis, CA) was applied as a pretreatment for 5h at 20 mM. Diphenylene iodonium (DPI, Alexis Biomedicals, Lansen, CH) was applied as a pre-treatment at 20µM for 1h. AG1478 and AG1296 were applied as a pre-treatment at 2µM for 2h. FTI-277 (Calbiochem, San Diego, CA) was applied at 20µM for 2 h. PD98059 (Calbiochem, San Diego, CA) was applied at 40µM for 2h. IL2 was applied at 1000 UI/ml for 15 min, INFγ was applied at 500 UI/ml for 15 min, TGFβ was applied at 20ng/ml for 15 min, 4- (2- aminoethyl benzenesulfonylfluoride (AEBSF, sigma, St. Louis, CA) was applied as a pre-treatment for 15 min at 40 µM and H2O2 was applied for 15 min at 2mM.

### EXAMPLES

Results of various experiments are presented in Table I.

**Table I: Representative assays, in suitable cell systems, for detection of stimulatory molecules in biological fluids from autoimmune disease affected patients.**

| **Diseases** | Lung fibrosis | Multiple Sclerosis (CSF) | | | Multiple Sclerosis (serum IgG) | Rheumatoid Arthritis (Serum) | Systemic sclerosis | | Grave's Disease |
|---|---|---|---|---|---|---|---|---|---|
| **Cell types** | HumanLF Fibroblast | Mouse cortical neuron | Human Oligodendrocyte | Neuro-blastoma cell | Human Oligoden-drocyte | Human Fibroblast | Human Fibroblast | Mouse Fibroblast Fα | Human thyroid cell |
| | Exps **A** | Exps **B** | Exps **C** | Exps **D** | Exps **E** | Exps **F** | Exps **G** | Exps **H** | Exps **I** |
| **ROS 1** | + | | | | + | + | ++ | +++ | + |
| **RAS 2** | + | ++ | | | | + | ++ | | |
| **ERK¹⁰ 3** | + | + | + | ++ | | + | + | | + |
| **JNK 4** | | | ++ | | | | | | |
| **DUOX 5** | | | | | | | | | +++ |
| **α-SMA6** | +++ | | | | | | | | + |

| | | | | | | | | | |
|---|---|---|---|---|---|---|---|---|---|
| The letters indicate the cell type and the disease. The numbers (1,2,3 ..) in the first column indicate the specific assay. For example Exp A1 indicates the experiments measuring ROS in human fibroblasts derived from lung fibrosis patients, relative to normal controls (at least 5). + p< 0.05; ++ p< 0.01; +++ p<0.001 vs. normal controls. | | | | | | | | | |

### EXAMPLE 1. Fibroblasts (systemic sclerosis and lung fibrosis).

### Systemic sclerosis

Systemic sclerosis (scleroderma, SSC) is a disorder characterized by fibrosis of the skin and visceral organs. Fibroblasts have been used to identify the stimulatory antibodies to the PDGFR that are present in systemic sclerosis.

Fibroblast from systemic sclerosis patients produce excess of ROS and maintain active Ras-ERK1/2. These cells accumulate DNA damage, activate ROS-dependent genes and become prone to stress-induced apoptosis. Inhibition of ROS, Ras or ERK1/2 down-regulates the loop and restores the normal phenotype in systemic sclerosis fibroblasts. These data point to Ras a key sensor of cellular ROS and suggest a molecular tool for the diagnosis and therapy of systemic sclerosis.

Authors set up an integrated assay in normal fibroblasts, which have been exposed for 15 min to SSC immunoglobulins or to PDGF, consisting in the detection of Ha or Ki-Ras, DUOX, ROS, PDGFR and P-ERK1/2.

Assays were performed in duplicate in primary human fibroblasts. The specificity of the assay was demonstrated by inhibition of the signal in cells pre-treated with general ROS scavengers (N-acethyl cysteine) or NADPH oxidase inhibitors (DPI or ABSF or Apocynin). By the use of this assay the authors were able to purify single clonal antibodies from SSC patients.

Results are shown in Figures 1, 2, 3 and 4.

### Lung fibrosis

Lung fibrosis or idiopathic pulmonary fibrosis (IPF) is a progressive and fatal disease with a mean survival rate of 3 years from the time of diagnosis. The features of the disease are the presence of foci of fibroblasts, scarring tissue and loss of alveolar space. It is thought that the illness is an autoimmune disease.

By using the assay of the present invention on fibroblasts isolated from biopsies of affected subjects, the authors found that these cells contain high ROS levels and are activated by a stimulatory molecule(s) that increases ROS (Fig. 5), Ras (Table 1, first column).

Lung fibroblasts from IPF patients show increased α-SMA levels compared to normal lung fibroblasts (Fig. 6).

### EXAMPLE 2. Lymphocytes (systemic autoimmune diseases).

The same type of regulation of Ras-ROS-ERK1/2 was found in primary lymphocytes of normal human subjects.

Results are shown in Figures 7 and 8. Activated lymphocytes *in vivo* display higher Ras-ROS and P-ERK1/2 content, as consequence of cytokine over-stimulation (not shown). This was found in cells derived from systemic autoimmune disases patients, such as SLE (systemic lupus erythematosus).

### EXAMPLE 3. Thyroid cells (Graves' disease).

Graves' disease is a frequent autoimmune thyroid disease characterized by hyperthyroidism and exophtalmus. The patients synthesize stimulating antibodies to TSHR. Agonistic antibodies target inflammation to sites where are located cells expressing TSHR (thyrocytes, adypocytes). In the retrorbital cavity, adipocytes expressing TSHR bind antibodies and attract immune cells. Exophtalmus is caused by local inflammation and characterizes the phenotype of the disease.

Although anti-TSHR antibodies have been detected and purified ca. 20 years ago, the exact mechanism of action and the difference between anti-TSHR antibodies and TSH biological actions effects are not known.

To validate the assay in these cells, authors tested the serum of normal, Graves and Hashimoto disease patients (Table II). Hashimoto thyroiditis is an autoimmune disease which does not implicate agonistic TSHR antibodies.

**Table II: Demographic characteristics of patients (Graves and Hashimoto) and controls (N) subjects.**

| Group | N° | M/F | Age Y(range) | ROS | Ras | P-ERK1/2 |
|---|---|---|---|---|---|---|
| **N** | 5 | 6/4 | 30-50 | 1 | 1 | 1 |
| **Graves** | 5 | 3/2 | 40-50 | 1.5±0.2 | 2 | 3 |
| **Hashimoto** | 4 | 1/3 | 30-50 | 1±0.2 | 1 | 1 |

| | | | | | | |
|---|---|---|---|---|---|---|
| Immunoglobulins were extracted from the patients indicated above. The diagnosis of the specific disease (Hashimoto or Graves' diseases) was defined according to stringent clinical and lab test criteria. The value 1 represent the level of Ras, ERK1-2 phosphorylated and ROS found in 5 normal controls. The methods were described in references 1 and 2. | | | | | | |

IgG from Graves patients induce ROS and maintain active Ras-ERK1/2 (Figures 9, 10). Dual NADPH oxidase/peroxidase (DUOX) proteins have recently been identified as components of the hydrogen peroxide generation system of the thyroid. The figure 11 shows that IgGs from Graves' patients induced DUOX protein levels in human thyroid cells.

### EXAMPLE 4. Neural and oligondendroglial cells (Multiple Sclerosis).

Multiple Sclerosis (MS) is a chronic disease diagnosed primarily in adults between the ages of 20 and 50. It is caused by the inflammation and scarring (sclerosis) of the myelin sheath and the underlying nerve due to an inflammatory process. The destruction of myelin seems to be due to an abnormal response of the immune system in which cells that normally protect against pathogens react against the body's own tissues. Exposure to a virus or other toxic or infectious agent in childhood might be the trigger for this abnormal autoimmune response.

Currently available treatments are based on disarming the immune system (T and B cells) either non-specifically (steroids-azatioprin) or specifically with anergy-inducing peptides (copaxone) or anti-VLA4 antibodies (Tsabiri).

Authors propose an alternative and novel approach, that will be useful for early diagnosis and staging of MS as well as a source of a new therapeutic tool by identification of specific agonistic antibodies that reacts and over-stimulate oligodendrocitic (myelin synthesizers) or neuronal cells by excess of ROS and maintain active Ras-ERK1/2. The assay will also detect other positive signalling molecule present in the serum or cerebrospinal fluid of MS patients. Over-stimulation will lead to selective oligondendroglial cells and loss of myelin synthesizing activity.

Indeed, it was observed that CSF from multiple sclerosis patients induce Ha-Ras and Ki-Ras protein levels (figs. 12 and 13) and P-ERK1/2 (fig. 14) in mouse cortical neurons. Moreover, serum IgG from Multiple Sclerosis Patients increases ROS levels in human oligodendrocytes (fig. 15).

It was also found that CSF from Multiple Sclerosis patients induce P-ERK1/2 and P-JNK levels in human oligodendrocytes (fig. 16 and 17).

CSF from Multiple Sclerosis patients also induces P-ERK1/2 in human neuroblastoma cells (fig. 18).

### EXAMPLE 5. Human skin fibroblasts (Rheumatoid arthritis).

Rheumatoid arthritis is a systemic disease that affects the synovial joints. It is a persistent chronic disease that spreads from joint to joint and affects about 0.5% of people worldwide. It typically develops in middle or late life and is three times more common in women than it is in men. It is characterised by joint pain, swelling, and stiffness, particularly of the small joints of the hands and feet. Its onset varies between gradual and acute. Current available treatment options comprise analgesics, non-steroidal inflammatory drugs, disease modifying drugs such as methotrexate, steroids, and the new biologicals (for example, anti-tumour necrosis factor agents).

The authors of the present invention found that serum from rheumatoid arthritis patients induces ROS levels in human skin fibroblasts (fig. 19).

### Loop linking an activated receptor to ROS-Ras expression.

The diagram of fig. 20 shows the core of the assay. Any type of receptor, when activated, stimulates directly or indirectly NADPH oxidase (nox2) or dual specificity NADPH oxidase (DUOX), resulting in ROS production and Ras stabilization (reference 2).

The assay described in the present invention permits the detection and isolation of stimulatory molecules present in biological fluids and the diagnosis of autoimmune diseases such as multiple sclerosis, lung fibrosis, rheumatoid arthritis, Graves' disease or systemic sclerosis. The assay works for any type of receptor because is based on the intimate link between energy metabolism (NADPH oxidase) and signal transduction. As proof of concept, the identification of stimulatory antibodies targeting PDGFR in systemic sclerosis and TSH agonistic antibodies in Graves' disease have been presented.

### REFERENCES

1. Seru R, Mondola P, Damiano S, Svegliati S, Agnese S, Avvedimento EV, Santillo M. HaRas activates the NADPH oxidase complex in human neuroblastoma cells via extracellular signal-regulated kinase 1/2 pathway. J Neurochem. 2004; 91(3):613-22.
2. Svegliati Baroni SS, Cancello R, Sambo P, Lucchetti M, Paroncini P, Orlandini G, Discepoli G, Patemo R, Santillo M, Cuozzo C, Cassano S, Avvedimento VE, Gabrielli A. PDGF and reactive oxygen species (ROS) regulate Ras protein levels in primary human fibroblasts via ERK1/2. Amplification of ROS and Ras in systemic sclerosis fibroblasts. J Biol Chem. 2005; 280(43):36474-82.
3. Svegliati Baroni SS, Santillo M, Bevilacqua F, Lucchetti M , Spadoni T., Mancini, M., Fraticelli P., Sambo P, Funaro, A.,Kazlauskas, A., Avvedimento VE*, Gabrielli A*. Stimulatory autoantibodies to the PDGF receptor in systemic sclerosis (scleroderma). N Engl J Med. 2006; 354(25):2667.
4. Andrews A, Balciunaite E, Leong FL et al. Platelet-derived growth factor plays a key role in proliferative vitreoretinopathy. Invest Ophthalmol Vis Sci. 1999; 40: 2683-9.
5. F. Curcio, F.S. Ambesi-Impiombato, G. Perrella and H.G. Coon. Long-term culture and functional characterization of follicular cells from adult normal human thyroids. Proc Natl Acad Sci USA, 1994; 91, 9004.
6. Boast S et al. Functional analysis of cis-acting DNA sequences controlling transcription of the human type I collagen genes JBC, 1990; 265, 13351.
7. J R de Wet, K V Wood, M DeLuca, D R Helinski, and S Subramani. Firefly luciferase gene: structure and expression in mammalian cells. Mol Cell Biol. 1987;7(2), 725-37.
8. Feliciello et al. Expression of thyrotropin-receptor mRNA in healthy and Graves' disease retro-orbital tissue. Lancet 1993; 342, 318.

## Claims

1. A method for the diagnosis and/or prognosis of an autoimmune disease or to monitor the efficacy of a therapy for an autoimmune disease in a subject comprising:
a) incubating under appropriate conditions a suitable cell system with a suitable amount of a biological sample drawn from the subject;
b) measuring the variation of more than one molecular species in said suitable cell system with respect to a proper control, said molecular species belonging to the following group: ROS, Ha-Ras, Ki-Ras, ERK1/2, JNK, DUOX and α-SMA,
wherein the autoimmune disease is selected from the group of Graves' disease or rheumatoid arthritis and
- when the autoimmune disease is Graves' disease, the biological sample is blood serum and the suitable cell system is a thyroid derived cell and
- when the autoimmune disease is rheumatoid arthritis, the biological sample is blood serum and the suitable cell system is a fibroblast cell system.

2. The method according claim 1 wherein the suitable cell system is genetically modified to express a detectable marker under the control of a promoter, said promoter being inducible by one of the molecular species as in claim 1.

3. The method according to claim 2 wherein the detectable marker is a luciferase.

4. The method according to claim 2 or 3 wherein the promoter is the collagen alpha 2 promoter.

5. A method of screening for autoimmune disease therapeutic agents comprising:
a) incubating under appropriate conditions a suitable cell system with a suitable amount of a biological sample drawn from an affected subject in the presence of a suitable amount of the putative autoimmune disease therapeutic agent;
b) measuring the decrease of more than one molecular species in said suitable cell system with respect to a proper control, said molecular species belonging to the following group: ROS, Ha-Ras, Ki-Ras, BRK1/2, JNK, DUOX and α-SMA,
wherein the autoimmune disease is selected from the group of Graves' disease, systemic sclerosis or rheumatoid arthritis and
- when the autoimmune disease is Graves' disease, the biological sample is blood serum and the suitable cell system is a thyroid derived cell;
- when the autoimmune disease is systemic sclerosis, the biological sample is blood serum and the suitable cell system is a fibroblast cell system or a Fα fibroblast;
- when the autoimmune disease is rheumatoid arthritis, the biological sample is blood serum and the suitable cell system is a fibroblast cell system.

6. The method according to claim 5 wherein the suitable cell system is genetically modified to express a detectable marker under the control of a promoter, said promoter being inducible by one of the molecular species as in claim 5.

7. The method according to claim 6 wherein the detectable marker is a luciferase.

8. The method according to claim 6 or 7 wherein the promoter is the collagen alpha 2 promoter.

## Patentansprüche

1. Verfahren zur Diagnose und/oder Prognose einer Autoimmunkrankheit oder zum Kontrollieren der Wirksamkeit einer Therapie gegen eine Autoimmunkrankheit bei einem Individuum, umfassend:
a) Inkubieren eines geeigneten Zellsystems unter angemessenen Bedingungen mit einer geeigneten Menge einer aus dem Individuum entnommenen biologischen Probe;
b) Messen der Variation von mehr als einer Molekülspezies in dem geeigneten Zellsystem in Bezug auf eine ordnungsgemäße Kontrolle, wobei die Molekülspezies zu der folgenden Gruppe gehört: ROS, Ha-Ras, Ki-Ras, ERK1/2, JNK, DUOX und α-SMA,
wobei die Autoimmunkrankheit aus der Gruppe Basedow-Krankheit oder rheumatoide Arthritis ausgewählt ist und
- wobei es sich bei der Autoimmunkrankheit um die Basedow-Krankheit, bei der biologischen Probe um Blutserum und bei dem geeigneten Zellsystem um eine Zelle aus der Schilddrüse handelt und
- wobei es sich bei der Autoimmunkrankheit um rheumatoide Arthritis, bei der biologischen Probe um Blutserum und bei dem geeigneten Zellsystem um ein Fibroblastenzellsystem handelt.

2. Verfahren nach Anspruch 1, wobei das geeignete Zellsystem genetisch modifiziert ist, um einen detektierbaren Marker unter der Kontrolle eines Promotors zu exprimieren, wobei der Promotor durch eine der Molekülspezies nach Anspruch 1 induzierbar ist.

3. Verfahren nach Anspruch 2, wobei es sich bei dem detektierbaren Marker um eine Luziferase handelt.

4. Verfahren nach Anspruch 2 oder 3, wobei es sich bei dem Promotor um den Kollagen-alpha2-Promotor handelt.

5. Verfahren zum Durchmustern auf therapeutische Mittel gegen eine Autoimmunkrankheit, umfassend:
a) Inkubieren eines geeigneten Zellsystems unter angemessenen Bedingungen mit einer geeigneten Menge einer aus einem betroffenen Individuum entnommenen biologischen Probe in der Gegenwart einer geeigneten Menge eines putativen therapeutischen Mittels gegen eine Autoimmunkrankheit;
b) Messen der Verringerung von mehr als einer Molekülspezies in dem geeigneten Zellsystem in Bezug auf eine ordnungsgemäße Kontrolle, wobei die Molekülspezies zu der folgenden Gruppe gehört: ROS, Ha-Ras, Ki-Ras, ERK1/2, JNK, DUOX und α-SMA,
wobei die Autoimmunkrankheit aus der Gruppe Basedow-Krankheit, systemische Sklerose oder rheumatoide Arthritis ausgewählt ist und
- wobei es sich bei der Autoimmunkrankheit um die Basedow-Krankheit, bei der biologischen Probe um Blutserum und bei dem geeigneten Zellsystem um eine Zelle aus der Schilddrüse handelt;
- wobei es sich bei der Autoimmunkrankheit um systemische Sklerose, bei der biologischen Probe um Blutserum und bei dem geeigneten Zellsystem um ein Fibroblastenzellsystem oder einen Fα-Fibroblasten handelt;
- wobei es sich bei der Autoimmunkrankheit um rheumatoide Arthritis, bei der biologischen Probe um Blutserum und bei dem geeigneten Zellsystem um ein Fibroblastenzellsystem handelt.

6. Verfahren nach Anspruch 5, wobei das geeignete Zellsystem genetisch modifiziert ist, um einen detektierbaren Marker unter der Kontrolle eines Promotors zu exprimieren, wobei der Promotor durch eine der Molekülspezies nach Anspruch 5 induzierbar ist.

7. Verfahren nach Anspruch 6, wobei es sich bei dem detektierbaren Marker um eine Luziferase handelt.

8. Verfahren nach Anspruch 6 oder 7, wobei es sich bei dem Promotor um den Kollagen-alpha2-Promotor handelt.

## Revendications

1. Procédé de diagnostic et/ou de pronostic d'une maladie auto-immune ou de surveillance de l'efficacité d'une thérapie contre une maladie auto-immune chez un sujet, ledit procédé comprenant :
a) l'incubation dans des conditions appropriées d'un système cellulaire adapté avec une quantité adaptée d'un échantillon biologique prélevé sur le sujet ;
b) la mesure de la variation de plus d'une espèce moléculaire dans ledit système cellulaire adapté relativement à un témoin adéquat, lesdites espèces moléculaires appartenant au groupe suivant : ROS, Ha-Ras, Ki-Ras, ERK1/2, JNK, DUOX et α-SMA,
dans lequel la maladie auto-immune est sélectionnée dans le groupe de la maladie de Graves ou de la arthrite rhumatoïde et
- lorsque la maladie auto-immune est la maladie de Graves, l'échantillon biologique est du sérum sanguin et le système cellulaire adapté est une cellule dérivée de la thyroïde et
- lorsque la maladie auto-immune est la arthrite rhumatoïde, l'échantillon biologique est du sérum sanguin et le système cellulaire adapté est un système cellulaire fibroblastique.

2. Procédé selon la revendication 1, dans lequel le système cellulaire adapté est génétiquement modifié pour exprimer un marqueur détectable sous le contrôle d'un promoteur, ledit promoteur étant inductible par l'une des espèces moléculaires selon la revendication 1.

3. Procédé selon la revendication 2, dans lequel le marqueur détectable est une luciférase.

4. Procédé selon la revendication 2 ou 3, dans lequel le promoteur est le promoteur du collagène alpha 2.

5. Procédé de criblage d'agents thérapeutiques contre une maladie auto-immune, ledit procédé comprenant :
a) l'incubation dans des conditions appropriées d'un système cellulaire adapté avec une quantité adaptée d'un échantillon biologique prélevé sur un sujet affecté en présence d'une quantité adaptée de l'agent thérapeutique putatif contre la maladie auto-immune ;
b) la mesure de la réduction de plus d'une espèce moléculaire dans ledit système cellulaire adapté relativement à un témoin adéquat, lesdites espèces moléculaires appartenant au groupe suivant : ROS, Ha-Ras, Ki-Ras, ERK1/2, JNK, DUOX et α-SMA,
dans lequel la maladie auto-immune est sélectionnée dans le groupe de la maladie de Graves, de la sclérodermie généralisée et de la arthrite rhumatoïde et
- lorsque la maladie auto-immune est la maladie de Graves, l'échantillon biologique est du sérum sanguin et le système cellulaire adapté est une cellule dérivée de la thyroïde ;
- lorsque la maladie auto-immune est la sclérodermie généralisée, l'échantillon biologique est du sérum sanguin et le système cellulaire adapté est un système cellulaire fibroblastique ou un fibroblaste Fα ;
- lorsque la maladie auto-immune est la arthrite rhumatoïde, l'échantillon biologique est du sérum sanguin et le système cellulaire adapté est un système cellulaire fibroblastique.

6. Procédé selon la revendication 5, dans lequel le système cellulaire adapté est génétiquement modifié pour exprimer un marqueur détectable sous le contrôle d'un promoteur, ledit promoteur étant inductible par l'une des espèces moléculaires selon la revendication 5.

7. Procédé selon la revendication 6, dans lequel le marqueur détectable est une luciférase.

8. Procédé selon la revendication 6 ou 7, dans lequel le promoteur est le promoteur du collagène alpha 2.
